# EUROPEAN PATENT APPLICATION

(11) **EP 1 488 698 A1**
(43) Date of publication of application: **22.12.2004**
(21) Application number: 04021743.2
(22) Date of filing: 23.12.1996
(51) Int. Cl.: A01N 43/04, A01N 57/26, A01N 33/02, A61K 31/70, A61K 31/685, A61K 31/13

(54) **Compositions for descreasing intracellular inositol trisphosphate concentration and uses thereof**

(30) Priority: 21.12.1995 US 9007 P
(62) Divisional of application: 96945258.0
(71) Applicant: Bio-Ved Pharmaceuticals, Inc., San Jose, CA 95112 (US)
(72) Inventor: The designation of the inventor has not yet been filed
(74) Representative: VOSSIUS & PARTNER

(57) **Abstract**

Compositions and methods for the modulation of aberrant inositol trisphosphate concentration in neurons is disclosed. Inositol trisphosphate stimulates an increase in intraneuronal calcium ion concentration which can, if unregulated, lead to acute neurotoxicities following such insults as brain seizures, and brain anoxia/ischemia; and lead to chronic neurotoxicities in such diseases as Alzheimer's disease, Huntington's disease Parkinson's disease, and amyotrophic lateral sclerosis. Inhibited inositol trisphosphate productions results in aberrantly low calcium ion levels leading to neuronal apoptosis.

## Description

### Background of the Invention

This invention relates to modulation of inositol trisphosphate (InsP₃) concentration in neurons.

Within the nervous system, information is conveyed from one neuron to another by electrical signals that are generated by the flux of ions, including calcium ions, across the neuronal cell membrane. When certain cell surface receptors are bound, calcium enters the cell through selective channels and may also be released from intracellular stores. The cell surface receptors involved include those that are bound by excitatory amino acids such as glutamate. Glutamate, and other agonists (discussed below), bind metabotropic receptors that are coupled to G proteins, and thereby instigate the biochemical cascade that leads to the release of calcium from intracellular stores.

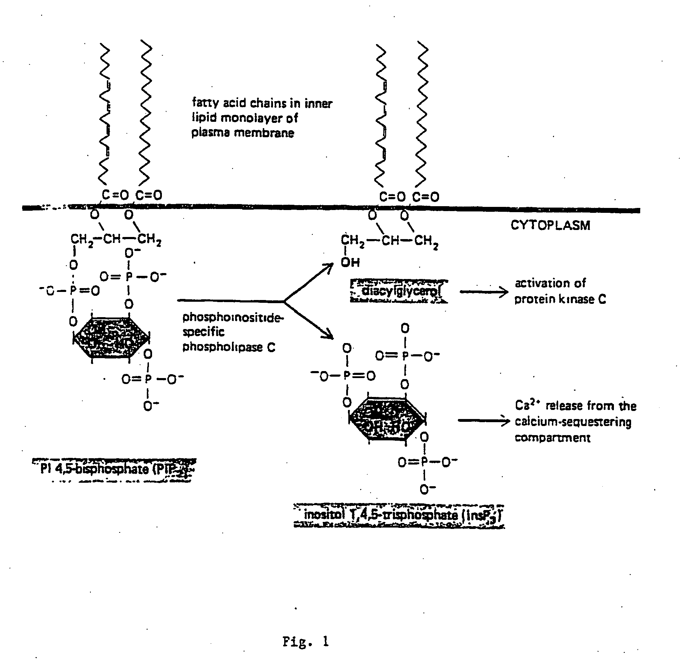

There are seven immunologically distinct subtypes of metabotropic glutamate receptors (M1 - M7). When bound, two of these receptor subtypes, M1 and M5, produce the second messenger InsP₃ by stimulating phosphoinositide-specific phospholipase C (hereinafter, "phospholipase C"), which converts phosphatidylinositol bisphosphate, a lipid located in the plasma membrane, to diacylglycerol and InsP₃ (this reaction is illustrated in Fig. 1).

In addition to L-glutamate, metabotropic receptors are activated by L-aspartate and by the pharmacological agonists quisqualate, ibotenate, and *trans*-ACPD (*trans*-(+-1)-1-amino-1,3-cyclopentanedicarboxylate; Schoepp *et al.*, Trends Pharmacol. Sci., 11:508-515, 1990). L-aspartate and aspartate analogs also act as agonists for metabotropic receptors expressed by neurons in the brain (Porter *et al.*, Neurosci. Lett., 144:87-89, 1992). In addition to stimulating metabotropic receptors, quisqualate and ibotenate stimulate ionotropic receptors, which are coupled to ion channels (Watkins *et al*., Trends Pharmacolo. Sci. 11:25-33, 1993). Thus, of the excitatory amino acid receptor agonists, *trans*-ACPD may be more selective for phosphoinositide-linked metabotropic receptors (Desai and Conn, Neurosci. Lett., 109:157-162. 1990). Pharmacological testing has also shown that L-trans-pyrrolidine-2,4-dicarboxylate and D,L-homocysteate stimulate receptor-coupled phosphoinositide hydrolysis in rat brain tissue (Li and Jope, Biochem. Pharmacol. 38:2781-2787, 1989).

Overstimulation of metabotropic receptors is thought to occur in the course of several neurological disorders. This overstimulation, and the resulting increase in InsP₃ production, increases intracellular calcium to levels that produce severe hyper-functional defects (see, for example, Thomsen *et al*., J. Neurochem., 62:2492-2495, 1994) and eventual neurotoxicity and death (Berridge, Nature, 361:315-325, 1993; Choi and Rothman, Ann. Rev. Neurosci., 13:171-182, 1990). Specific disorders associated with overstimulation of metabotropic glutamate receptors in the brain include limbic seizures (Tiziano *et al*., Neurosci. Lett., 162:12-16, 1993) and chronic neurodegenerative disorders such as Alzheimer's disease, Huntington's disease, Parkinson's Disease, and amyotrophic lateral sclerosis (ALS; more commonly known as Lou Gehrig's Disease).

Another type of neuronal cell death, referred to as programmed cell death or apoptosis, may be affected by reduced activity of metabotropic glutamate receptors (Copani *et al*., J. Neurochem. 64:101-108, 1995). Similarly, inhibition of InsP₃ is thought to mediate neuronal apoptosis by reducing intracellular calcium.

### Summary of the Invention

The work described herein is aimed at altering the mechanism(s) that regulate the concentration of InsP₃ in order to treat neurological disorders that are associated with hyperactive function and neuronal cell death. Accordingly, the present invention provides methods and compositions for modulating the concentration of InsP₃ in neurons. Disorders associated with glutamate excitotoxicity (either directly or indirectly) should be particularly amenable to treatment with these compositions and methods.

### Brief Description of the Drawing

Fig. 1 is a diagram illustrating conversion of phosphitidyl inositol 4,5,-bisphosphate (PIP₂) into diacylglycerol and inositol trisphosphate (InsP₃).

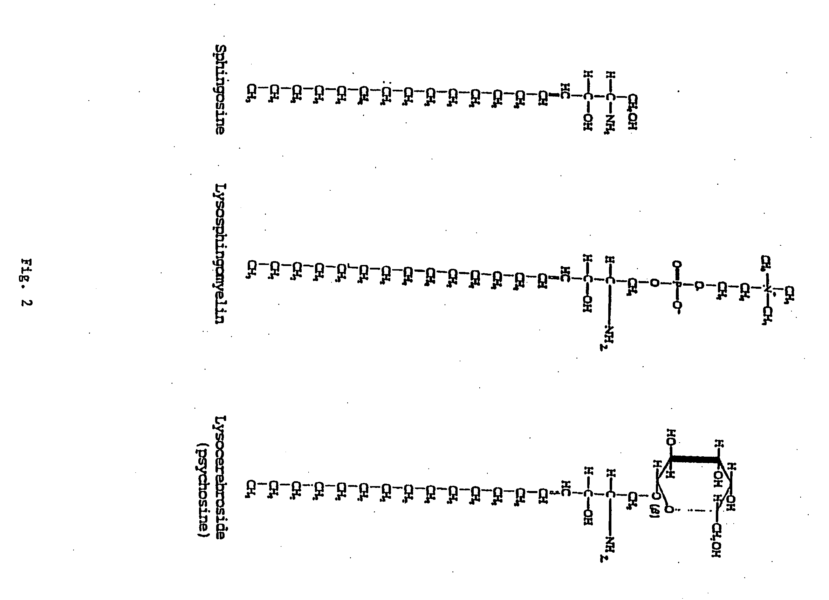

Fig. 2 is a diagram of the structure of the (lyso) sphingolipids sphingosine, lysosphingomyelin, and lysocerebroside.
Fig. 3 is a bar graph illustrating the effects of sphingosine and psychosine (lysocerebroside) on basal and bradykinin-stimulated phosphoinositide signalling in PC-12 cells. The bars marked A represent cells that were untreated. The bars marked B represent cells that were exposed to 10 µM bradykinin for 30 minutes. The bars marked C represent cells that were exposed to 100 µg/ml sphingosine for 30 minutes before treatment with an excitotoxic agent.
Fig. 4 is a graphical representation of the concentration effects of psychosine repression of bradykinin-stimulated phosphoinositide signalling in PC12 cells.
Figs. 5A and 5B are scanned images at low (Fig. 5A) and high power magnification (Fig. 5B) of the CA1 region of the hippocampus 7 days after infusion of 225 nm quisqualate.
Figs. 6A and 6B are scanned images at low (Fig. 6A) and high power magnification (Fig. 6B) of the CA1 region of the hippocampus 7 days after infusion of saline.
Figs 7A and 7B are scanned images at low (Fig. 7A) and high power magnification (Fig. 7B) of the CA1 region of the hippocampus seven days after treatment with 125 nm psychosine and subsequent exposure to 225 quisqualate.
Fig. 8 is a bar graph illustrating the mean number of convulsions or spasms (±SEM) across four groups of treated animals. Q = quisqualate; P = psychosine; SP = lysosphingomyelin.
Fig. 9 is a bar graph illustrating the mean duration (in minutes) of three behaviors (±SEM): teeth chatter, akinesia, and mobilizing. Q = quisqualate; P = psychosine; SP = lysosphingomyelin.

### Detailed Description

Sphingolipid is the name given to derivatives of fatty acid-containing compounds of the long chain amphiphilic amino alcohol sphingosine whose terminal hydroxyl group is substituted by phosphoryl, glycosyl, or other groups. Cationic sphingolipids that lack the fatty acid component of the parent sphingolipid compound are called lysosphingolipids. Free unsubstituted sphingosine and the terminally substituted derivatives of sphingosine: lysosphingomyelin (sphingosyl phosphorylcholine), lysocerebroside (also called glycosyl sphingosine or psychosine), lysosulphatides, and lysogangliosides are all lysosphingolipids.

It is disclosed herein that, at non-toxic physiological levels, naturally occurring sphingosine increases the production of InsP₃ in brain neurons, while naturally occurring lysosphingolipids, lysocerebroside, and lysosphingomyelin (and no other naturally occurring lysosphingolipids) potently repress InsP₃. It is further disclosed that lysosphingomyelin and lysocerebroside potently and specifically repress the increase in InsP₃ that is induced when neurons are exposed to excitatory amino acids or their analogue agonists, such as ibotenate and quisqualate. The repression of InsP₃ by lysosphingomyelin and lysocerebroside competes with the stimulation of InsP₃ by sphingosine.

The invention provides a method of modulating InsP₃ concentration in a neuronal cell of a mammal that has, or is suspected of having, a disorder associated with an abnormal concentration of InsP₃. The method involves administering to the mammal a composition containing an isolated compound that modulates the concentration of InsP₃ and a pharmaceutically acceptable carrier. The modulation produced by this method may result in a decrease in InsP₃ production (as can be caused by the compounds lysocerebroside or lysosphingomyelin) or an increase in InsP₃ production (as can be caused by the compound sphingosine). The carrier may consist of an excipient including buffers such as citrate buffer, phosphate buffer, acetate buffer, and bicarbonate buffer, amino acids, urea, alcohols, ascorbic acid, phospholipids, proteins such as serum albumin, gelatin, EDTA, sodium chloride, liposomes, polyvinylpyrollidone, mannitol, sorbitol, glycerol, propylene glycol, and polyethylene glycol (e.g., PEG-4000 or PEG-8000). The neuron that is contacted may reside within the peripheral nervous system or the central nervous system. Preferably, the neuron is within the brain.

The composition described above may be administered by any route known to skilled pharmacologists. The route of administration may be, for example, intra-arterial, intracerebral, intrapulmonary, or transmucosal. Preferably, administration is by subcutaneous, intramuscular, or intraperitoneal injection and, most preferably, by intravenous injection.

If necessary, the compounds of the invention, or compounds discovered by the method of the invention, can be modified to increase the efficiency with which they cross the blood brain barrier. In order to enable these compounds to penetrate the blood brain barrier, they can be delivered in encapsulated cell implants (e.g., those produced by CytoTherapeutics, Inc., Providence RI; see *Bioworld Today* 7:6, December 2, 1996). Delivery of drugs to the brain may also be accomplished using RMP-7™ technology (Alkermes, Inc., Cambridge, MA: see *Business* Wire, "Third Major Agreement for Prolease Sustained Release Drug Delivery System," (December 2, 1996) or implantable wafers containing the drug (see *PR Newswire*, "Implantable Wafer is First Treatment to Deliver Chemotherapy Directly to Tumor Site, " September 24, 1996). The compositions may also be administered using an implantable pump for direct administration into intrathecal fluid (e.g., that made by Medtronic, Minneapolis, MN; see *Genetic Engineering News,* "Neurobiotechnology Companies Focus Programs on Pain and Neuroprotection," November 1, 1996).

The route of administration and the amount of protein delivered will be determined by factors that are well within the ability of skilled artisans to assess. Furthermore, skilled artisans are aware that the route of administration and dosage of a therapeutic substance may be varied for a given patient until a therapeutic dosage level is obtained. The dosage and length of any treatment are known to depend on the nature and severity of the disease and to vary from patient to patient as a function of age, weight, sex, and general health, as well as the particular compound to be administered, the time and route of administration and other drugs being administered concurrently. Skilled artisans will be guided in their determination of the appropriate therapeutic regime by, e.g., Gregoriadis (*Drug Carriers in Biology and Medicine,* Academic Press) and Goodman and Gilman (*The Pharmacological Basis of Therapeutics,* 6th Edition). Skilled artisans can be guided further in their determination of the correct therapeutic dosage by assessing behavioral criteria, as disclosed in Example VIII, or by performing any standard test of a patient's cognitive or motor skills. Typically, the dosage of an InsP₃ modulatory substance described herein will range from 0.01 to 100 mg/kg of body weight. More preferably, sphingolipids such as sphingosine, lysosphingomyelin, and lysocerebroside are administered in the range from about 0.5 mg/kg body weight to 1.5 mg/kg body weight for each compound. It is expected that regularly repeated doses of the InsP₃ modulatory compound will be necessary over the life of the patient. Alternatively, a neuron may be contacted *in vitro.*

A pharmaceutical composition containing a compound that modulates InsP₃ in mawmalian neuronal tissue and a pharmaceutically acceptable carrier is another embodiment of the invention. This composition can modulate the concentration of InsP₃ by either increasing or decreasing the concentration of InsP₃ within a neuron, such as a neuron within the brain, to a concentration that is sufficient to treat a disorder that is associated with abnormal InsP₃ production.

When an increase in intracellular InsP₃ is sought, the isolated compound of the invention is a lysosphingolipid which may be, but is not limited to, sphingosine. When a decrease in intracellular InsP₃ is sought, the isolated compound of the invention is a lysosphingolipid which may be, but is not limited to, lysosphingomyelin or lysocerebroside. Preferably, the mechanism by which the composition of the invention modulates inositol trisphosphate concentration is by modulating the activity of phosphoinositidase-specific phospholipase C. The composition can be used to modulate InsP₃ in a neuron *in vitro* or *in vivo*.

When the invention provides a composition containing a lysosphingolipid in a pharmaceutically acceptable carrier, the lysosphingolipid is preferably characterized as being the D-erythro isomer and having: (1) a *trans*-4 double bond, (2) a net positive charge, (3) an aliphatic chain of 8 or more carbon atoms linked in series, and (4) a net neutrally charged substituent on the oxygen atom of carbon atom 1. The substituent may be, but is not limited to, any of the following: hydrogen, monosaccharide, disaccharide, trisaccharide, polysaccharide, phosphorylcholine, and phosphoryl ethanolamine. Preferably, agents fitting these parameters and occurring at physiological, ng/mg protein levels (Kolesnick, J. Biol. Chem., 264:7617, 1989), are the naturally occurring lysosphingolipids sphingosine, psychosine, and lysosphingomyelin. Saturation of the double bond of sphingosine by hydrogenation produces the compound sphinganine which has less than one third the membrane insertion potential of sphingosine. Compounds such as N-acetyl sphingosine or ceramide, in which the amino group is amidated and rendered non-ionic, apparently have little or no physiological effect in this system. A cationic free base, 4-trans-enic amphiphile provides physiological activity. Ionically neutral 1-0-substitution provides inhibitory activity.

A method of identifying a compound that modulates the production of InsP₃, preferably in a neuron, is also provided by the invention. In this method, a neuron is contacted, either *in vitro* or *in vivo* with a compound and inositol, such as tritium-labeled inositol, under conditions sufficient to modulate InsP₃ production. The amount of inositol trisphosphate produced is then measured. Any change in the concentration of InsP₃ can be measured by standard techniques known to one of ordinary skill in the art. For example, one can monitor the amount of radiolabelled InsP₃ produced from prelabelled inositol starting material. Alternatively, symptoms of a mammal having a neural disorder can be monitored. The compound identified by this method of the invention may modulate an increase or a decrease in InsP₃ production. The method of identifying a compound that modulates neuronal inositol trisphosphate concentration may also include contacting the neuron with a second compound that modulates InsP₃.

The invention also provides for a first and a second compound contacting a neuron under conditions sufficient to modulate InsP₃ production.

In addition to neurons, the nervous system contains neuroglia, such as astrocytes, Schwann cells, and microglia, which contribute to repair processes in the nervous system and lend support to the neurons. Neuroglia and neurons are phenotypically distinct cell types. For example, astrocytes have major adrenergic (rather than excitatory amino acid) metabotropic receptors and, in these cells, InsP₃ production is enhanced by sphingosine and inhibited by psychosine (Ritchie *et al*., Biochem. Biophys. Res. Commun., 186:790-795, 1992). In addition, the way in which InsP₃ controls calcium ion concentration and signalling in astrocytes is distinctly different from that in brain neurons: in astrocytes, sphingolipid modulation of InsP₃ production occurs by α-adrenergic stimulation of InsP₃ production via a G protein intermediate that is inhibited by treatment with pertussis toxin (Ritchie *et al*., *supra*). Furthermore, InsP₃ produced in astrocytes is communicated through gap junctions to other cells, whereas in neurons, InsP₃ remains within the cell where it modulates the concentration of Ca²⁺.

Applicants have shown that sphingosine increases the basal level of InsP₃ in brain neurons independently of stimulation of excitatory amino acid receptors by any agonists (the effect of sphingosine being downstream from the receptors). Because the excitatory amino acid analogues are structurally and functionally unrelated to sphingolipids (as reflected by their action at a distinct point in the signalling pathway), these analogues do not predict the present invention.

The drug chlorpromazine (10-(3-dimethylaminopropyl)-2-chlorphenothiazine) raises the level of InsP₃ in rat C6 glioma cells (Leli and Hauser, Biochem. Biophys. Res. Commun., 135:465-472, 1986). However, chlorpromazine is unrelated structurally and functionally to the sphingolipid compositions of the present invention, and therefore does not predict the present invention.

Sphingosine and several sphingosine derivatives inhibit a protein kinase C isoform that is activated by diacylglycerol and phorbol esters in non-neuronal cell types such as lymphocytes, neutrophils, granulocytes, Chinese hamster ovary cells, and platelets. (Hannum and Bell, Trends Biochem. Sci., 20:73-77, 1987; and Grove and Maestro, Biochem. Biophys. Res. Commun., 151:94-99, 1988). However, it is disclosed herein that the effects of sphingosine on brain neurons are independent of protein kinase C inhibition: the protein kinase C inhibitor staurosporine does not influence the effects of sphingosine,. lysocerebroside, or lysosphingomyelin on InsP₃ production in brain neurons that are responsive to excitatory amino acids. As a result, the protein kinase C-mediated pathway and the phospholipase C-mediated pathway for controlling intraneuronal calcium ion concentration are independent. Consequently, compounds that affect the protein kinase C pathway are not predictive of the effects of compounds, such as the sphingolipids of the present invention, on phospholipase C pathway regulation.

A synthetic analog of the amino acid glycine ((S)-4-carboxy-3-hydroxyphenyl glycine) blocks a metabotropic (but not ionotropic) amino acid receptor and is thereby thought to render protection against seizures (Thomsen et *al*., *supra*). However, blockage of glutamate-responsive metabotropic receptors by therapeutic glutamate antagonists is highly impractical because these antagonists are generally toxic (Michel and Agid, J. Neurosci. Res., 40:764-775, 1995). In contrast, the compositions and methods of this invention contain naturally occurring lysosphingolipids that act downstream from the cell surface receptor. These substances are associated with a reduced risk of toxicity.

It is a significant advantage of the invention that naturally occurring compounds within a non-toxic physiologic range are used, limiting the possible dangers to the patient and undesirable side effects that accompany treatment with non-natural, synthetic pharmaceutical compounds. The lysophingolipids are metabolized and cannot accumulate to dangerous levels in patients, as is the case for the synthetic receptor blockers. Also, the lysosphingolipids used herein do not rely upon liver detoxification and kidney excretion so their use will have minimal risk of liver or kidney damage. In addition, receptor blockage disturbs normal brain function, which may be avoided by targeting phospholipase C and allowing receptor function to regulate neuronal ion balance. A further advantage of the invention is that the naturally occurring compounds of the invention are readily available, making use of the present invention potentially much less expensive to the patient than the use of synthetic pharmaceutical preparations.

An object of the invention is to provide methods and compositions that can be used to treat mammalian conditions associated with glutamate excitotoxity. These conditions include, but are not limited to, acute conditions stemming from brain anoxia or ischemia, brain seizure activity, and chronic conditions such as Alzheimer's disease, Parkinson's Disease, Huntington's disease, and amyotrophic lateral sclerosis.

Other features and advantages of the invention will be apparent from the following detailed description, and from the claims. Although methods and materials similar or equivalent to those described herein can be used in the practice or testing of the invention, the preferred methods and materials are described below.

The finding that lysosphingolipids influence InsP₃ production which, in turn, controls the concentration of calcium ions in neurons, led to the compositions and methods described herein for controlling aberrant InsP₃ production in various neuronal disorders.
The present invention is also characterized by the following items:
1. A method of modulating inositol trisphosphate concentration in a neuronal cell of a mammal having or suspected of having a disorder associated with an abnormal concentration of inositol trisphosphate, the method comprising:
   administering to the mammal a composition comprising an isolated compound which modulates inositol trisphosphate concentration by acting downstream from a cell surface receptor and a pharmaceutically acceptable carrier.
2. The method of item 1, wherein the modulation is a decrease in inositol trisphosphate production.
3. The method of item 1, wherein the compound is selected from the group consisting of lysocerebroside and lysosphingomyelin.
4. The method of item 1, wherein the modulation is an increase in inositol trisphosphate production.
5. The method of item 4, wherein the compound is sphingosine.
6. The method of item 1, wherein the contacting occurs *in vivo*.
7. The method of item 1, wherein the neuron is a central nervous system neuron.
8. The method of item 7, wherein the neuron is a brain neuron.
9. The method of item 1, wherein the neuron is a peripheral nervous system neuron.
10. A method of identifying a compound which modulates neuronal inositol trisphosphate concentration, the method comprising:
   a) contacting a neuron with inositol and a compound under conditions sufficient to modulate inositol trisphosphate production; and
   b) measuring the amount of inositol trisphosphate produced.
11. The method of item 10, wherein the compound modulates an increase in inositol trisphosphate.
12. The method of item 10, wherein the compound modulates a decrease in inositol trisphosphate.
13. The method of item 10, further comprising contacting the neuron with a second compound that modulates inositol trisphosphate concentration.
14. A pharmaceutical composition comprising:
   a) an isolated compound that modulates inositol trisphosphate concentration in mammalian neuronal tissue; and
   b) a pharmaceutically acceptable carrier.
15. The composition of item 14, wherein the modulation occurs i*n vivo*.
16. The composition of item 14, wherein the isolated compound modulates inositol trisphosphate concentration by modulating the activity of phosphoinositidase-specific phospholipase C.
17. The composition of item 14, wherein the compound is a sphingolipid.
18. The composition of item 17, wherein the sphingosine moiety of the sphingolipid is characterized as being the D-erythro isomeric form and having;
   a) a *trans*-4 double bond;
   b) a net positive charge;
   c) a substituent on the oxygen of carbon atom 1, wherein the substituent has a neutral net charge; and
   d) an aliphatic chain comprising 8 or more carbon atoms linked in series.
19. The composition of item 18, wherein the substituent on the oxygen of carbon atom 1 is selected from the group consisting of hydrogen, monosaccharide, disaccharide, trisaccharide, polysaccharide, phosphorylcholine, and phosphoryl ethanolamine.
20. The composition of item 14, wherein the composition modulates an increase in inositol trisphosphate production.
21. The composition of item 20, wherein the compound is sphingosine.
22. The composition of item 14, wherein the composition modulates a decrease in inositol trisphosphate concentration.
23. The composition of item 22, wherein the compound is selected from the group consisting of lysocerebroside and lysosphingomyelin.

Exemplary, non-limiting compositions and methods that can be used to carry out the invention are described below.

### Example 1: Preparation of Neuronal Cell Cultures

Primary cultures of neurons were prepared from the telencephalon of white Leghorn chick embryos on the eighth day of their development (Rosenberg *et al.*, J.Biol. Chem. 267:10601-10612, 1992). The neurons were seeded in 24-well plastic tissue culture plates that had been coated with L-polylysine. The density of the neurons was 10⁵ cells per well, and they were cultured in Dulbecco's modified Eagle's medium/Ham's high glucose F-12 medium (1:1, vol:vol) containing 500 ng/L sodium selenite, 500 µg/L transferrin, and 165 pmole/L EGF. The cultures were maintained at 37°C under 5% CO₂ in air for 4 days, by which time the cells had differentiated into neurite-bearing cortical granulocytic neurons. These cultures of mature embryonic neurons were used for phosphoinositide hydrolysis assays (as described in Example 2).

PC12 cells, from a rat adrenal pheochromocytoma cell line, were used as a model of neurons from the peripheral nervous system. These cells were obtained from a common laboratory cell culture stock and grown in plastic culture flasks in Dulbecco's modified Eagle's medium (DMEM) supplemented with 5% heat-inactivated horse serum and 10% heat-inactivated fetal calf serum (PC12 cells are also available from the American Type Culture Collection under the Accession number CRL 1721). The cells were grown at 37°C in 5% CO₂ in air until a confluent monolayer formed. The cells were dispersed into 24-well plastic tissue culture plates at a density of 10⁵ cells per well and used for the phosphoinositide hydrolysis assays (as described in Example 2).

### Example 2: Measurement of Inositol Trisphosphate Production by a Phosphoinositide Hydrolysis Assay

Receptor-stimulated and basal unstimulated hydrolysis of phosphoinositides was measured by the following procedure. In order to label the cellular phosphoinositides metabolically, the primary cultures of chick cortical neurons (described in Example 1) were incubated overnight with 0.5 ml Dulbecco's modified Eagle's medium containing 1 µCi [³H]myo-inositol per well. The cells were then washed twice with 1 ml Dulbecco's phosphate-buffered saline (PBS) and 0.5 ml of PBS containing 4.5 g/L glucose was added. Sphingosine, psychosine, lysosulphatide, or lysosphingomyelin, prepared as the hydrochloride salt dissolved in water, were then added to produce the desired extracellular concentration in specific wells, and the 24-well plates were swirled gently on a rotary shaker for 1 hour at 23°C. The cells were then washed twice with 1 ml PBS, and pre-incubated for 15 minutes at 37°C with 0.5 ml PBS containing 10 mM LiCl and the compound to be tested was added. After 30 minutes, the experiment was terminated by adding 1 ml of ice-cold methanol to each well and transferring the material in each well to polypropylene tubes that contained 0.4 ml water and 1 ml chloroform. The tubes were vortexed thoroughly, then centrifuged at 500 x g for 5 minutes to separate the aqueous and chloroform phases. For each sample, a 1.5-ml aliquot of the upper (aqueous) phase was applied to a small column containing BioRad AG1X8 resin (formate form). Free [³H]inositol and [³H]glycerophosphoinositol were washed through the column with 5 ml of a solution containing 5 mM sodium borate and 60 mM sodium formate. The total phosphorylated [³H] inositol fraction was eluted from the column for radiometric scintillation spectrometry analysis with 3 ml 1.0 M ammonium formate/0.1 M formic acid.

In addition, a 0.5 ml aliquot of the lower (chloroform) phase of each sample was analyzed radiometrically by scintillation spectrometry in order to determine the quantity of lipid-bound [³H]myo-inositol. Estimation of phosphoinositide hydrolysis by phospholipase C is based upon the quantity of [³H]inositol phosphates produced, and is expressed as the percentage of the latter relative to total free and componential [³H]inositol (dpm ammonium formate + dpm chloroform fractions).

### Example 3: InsP₃ Production in Neurons

### A. Inositol trisphosphate production in PC12 Cells

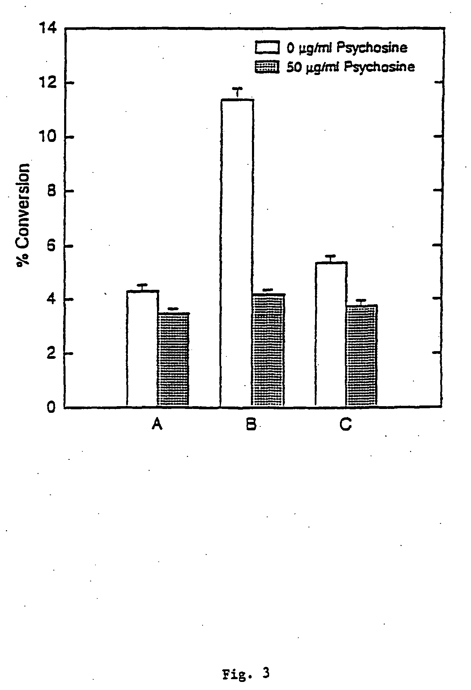

PC12 cells display a strong phospholipase C-linked metabotropic receptor response to bradykinin, an effective pain producing agonist. As with the other neuronal cells, sphingosine tonically upregulated the basal, unstimulated level of InsP₃ production in PC12 cells. Psychosine and lysosphingomyelin strongly inhibit InsP₃ production in PC12 cells and, even at relatively moderate levels, can entirely block the sensitivity of PC12 cells to bradykinin. These findings are shown in Fig 3.

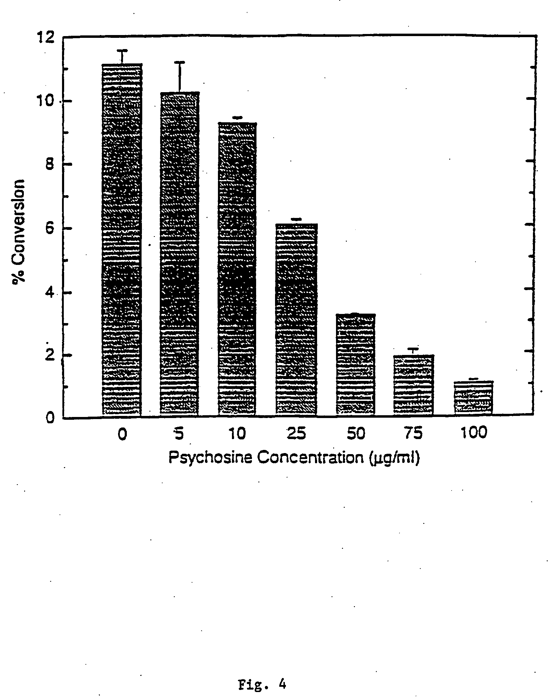

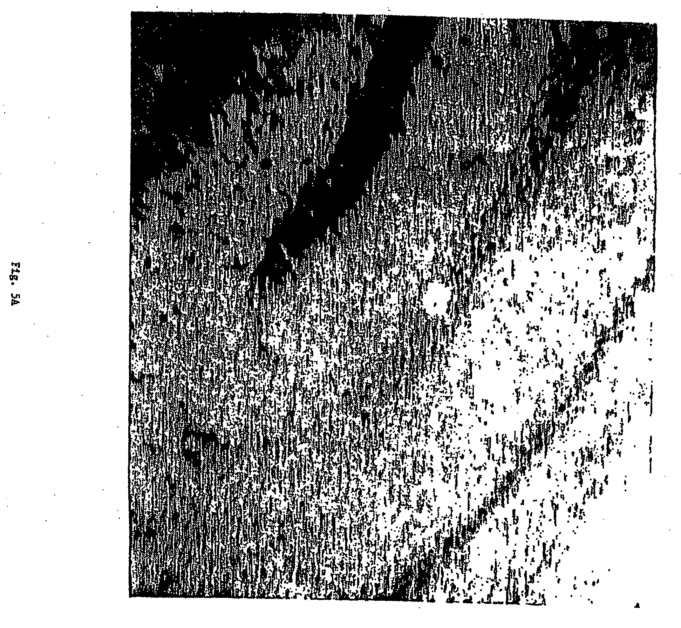

The degree of InsP₃ inhibition is a function of the log of the psychosine concentration for a fixed time of exposure (Fig. 4). Exposing PC12 cells to 50 µM exogenous psychosine for 30 minutes was sufficient to reduce a metabotropic response to 10 µM bradykinin to half maximum.

### B. InsP₃ Production in Cultured Neurons

Cultured primary chick cortical neurons that were prelabeled with [³H]myo-inositol were examined for enhanced InsP₃ signalling in response to various agonists including glutamate, aspartate, and the metabotropic M1/M5 receptor agonists ibotenate and quisqualate. As shown in Table 1, sphingosine, lysocerebroside, and lysosphingomyelin potently modulated InsP₃ signalling. Lysosulphatide was without inhibitory effect in this system. Thus, sphingosine tonically enhanced the basal, unstimulated InsP₃ level in cortical neurons, while psychosine and lysosphingomyelin blocked this enhancement whether it was caused by sphingosine or by metabotropic glutamate receptor agonists.

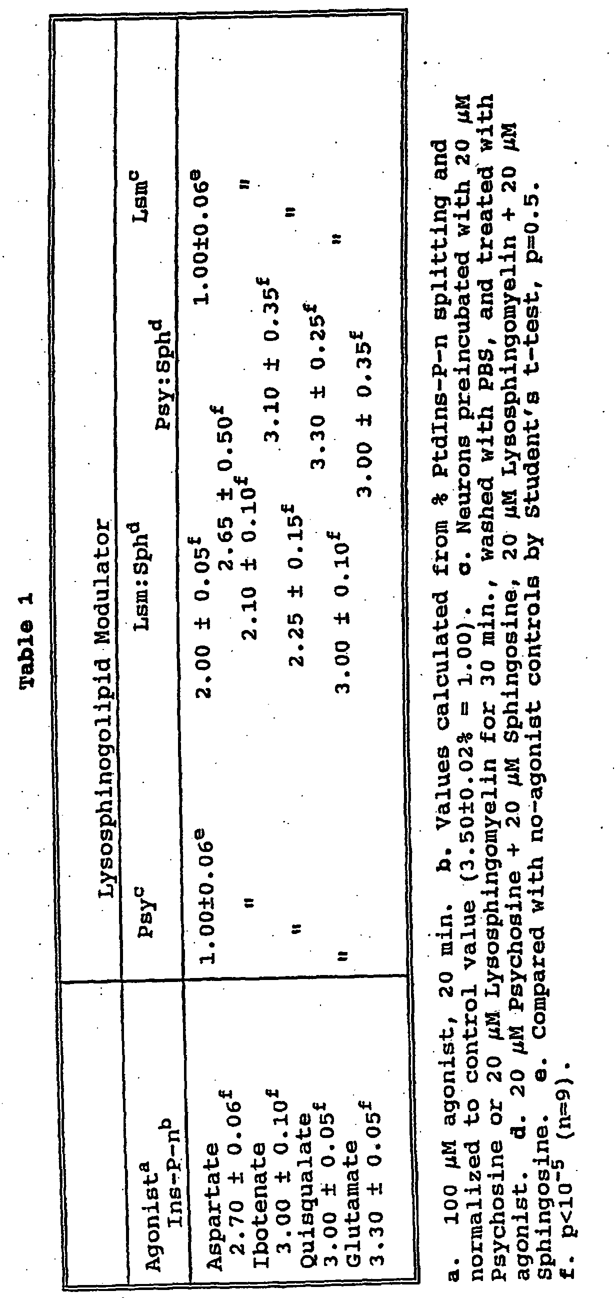

### Example 4: Monitoring Lysosphingolipid Content of Neurons

Agonist-binding to metabotropic receptors influences the levels of lysosphingolipid in neuronal membranes. However, there is no convenient methodology available for the measurement of lysosphingolipid content in cultured neurons. As a result, a sensitive method of fluorometric tracing has been devised and is described herein. The measurement of lysosphingolipid content in cultured neurons is based on the stable fluorescence-tagging procedure using 4-fluoro, 7-nitrobenzofuran (NBDZF) described by Nozowa *et al* J. Neutochem. 59:607-609, 1992). The procedure was applied to: resting neurons; neurons exposed to 100 µM metabotropic receptor agonist for 1 hour; resting neurons exogenously enriched in lysosphingolipids; and neurons exogenously enriched in lysosphingolipids and exposed to metabotropic receptor agonist for 1 hour. The neurons were scraped in batches of 10⁷ (1 Petri dish, approximately 8 cm in diameter, is equivalent to 2 mg membrane protein) and subjected to anaylsis.

Total lipids were removed from the neuron samples by repeated extraction with chloroform:methanol (2:1, vol:vol). The lysosphingolipids were separated from total neuronal lipid in 90 ± 5 % recovery yield (n = 10) by the stepped chromatographic procedure of Van Veldhoven et al. (Anal. Biochem. 183:177-189, 1989). The recovered lysosphingolipids were stably tagged with NBDZF by the rapid procedure of Nozowa *et al*. (*supra*). The fluorescence tagged neuronal lysosphingolipids were separated in parallel with reference standards NBDZF-Sph, NBDZF-Psy, and NBDZF-Lsm on high performance silica gel thin layer chromatography plates with acetonitrile:H₂O (10:0.5, vol:vol). The highly fluorescent, well separated bands were scraped from the plate. The fluorescence tagged lysosphingolipids are eluted from the scraped silica gel with methanol:1 N HCl (1:0.02, vol:vol). The fluorescence tagged lysosphingolipid eluates were quantitated against reference standards by fluorescence measurement in a Perkin-Elmer LS-5 Fluorescence Spectrometer.

Resting, unstimulated neurons contained 113 ± 13 pmoles sphingosine, 50 ± 10 pmoles lysocerebroside, and 5 ± 0.2 pmoles lysosphingomyelin per mg protein (n = 9). Neurons stimulated with L-glutamate agonist, e.g. 100 µM ibotenate for 1 hour contained 85 ± 3 pmoles sphingosine, 80 ± 5 pmoles lysocerebroside and 52 ± 2 pmoles lysosphingomyelin per mg protein (n = 6). It appears that a compensatory increase in the inhibitory lysosphingolipids may occur during extended exposure to agonist. Sphingosine is convertible in the cytoplasmic face of cis-Golgi (Burger and DeMeer, Trends Cell Biol., 2:332-337, 1992) to lysocerebroside by transfer of a glucosyl moiety from UDP-glucose to the 1-0-position of sphingosine by the action of glucosyltransferase:lysocerebroside (psychosine) synthase (Schwarzmann and Sandhoff, Methods in Enzymology, 138:319-341, 1987). An analogous system exists for lysosphingomyelin synthesis by transfer of a phosphorylcholine moiety from CDP-choline.

Neurons synthesize prodigious amounts of sphingolipids with 92 mass % of the total cellular sphingolipid located in the outer lipid bilayer of the plasma membrane. Sialoglyco-sphingolipids are present at the level of 40.8 ± 1.9 µg/mg cell protein, and sphingomyelin is present at 12.1 ± 3.0 µg per mg cell protein.

Sphingolipid content of neurons exposed to sphingosine, lysocerebroside, and lysosphingomyelin was examined as follows. A Petri dish containing approximately 10⁷ neurons was incubated with 5 µCi [³H]myo-Ins in 3 ml DMEM overnight to prelabel the phosphoinositide InsP₃ may contain. The neurons were washed with PBS and 3 ml of PBS containing 4.5 g/l glucose was added. Preparations of sphingosine, lysocerebroside, lysosphingomyelin (Sigma Chemical Co., St. Louis, MO) and combinations of each were prepared as the hydrochloride salt dissolved in PBS. The lysosphingolipids were added to the Petri dishes to produce a concentration ranging from 0 - 150 µM. The dishes were held at room temperature for 0.5, 1, or 2 hours, then washed with PBS and incubated with 5 Units of trypsin in glucose/PBS for 4 hours at 37°C. Trypsinization removes non-specifically bound lysosphingolipids that may adhere to extracellular plasma membrane protein domains. Non-specific adherence between cationic micelles of lysosphingolipids and the neuronal glycocalyx is avoided by performing the experiments with lysosphingolipid concentrations below the critical micelle concentration. Neurons were analyzed for lysosphingolipid content as described above.

Following exposure to 50 µM sphingosine for 1 hour, the content of sphingosine in the neurons was 430 ± 70 pmoles/mg protein (n = 9). Following exposure to 50 µM lysocerebroside, the content of sphingosine was 220 ± 45 pmoles/mg protein (n = 20), and exposure to 50 µM lysosphingomyelin resulted in 105 ± 0.15 pmoles sphingosine/mg protein (n = 9).

### Example 5: Monitoring Metabotropic Receptor Function

Metabotropic receptor function was examined in intact neurons by assaying lysosphingolipid modulation of calcium ion signalling. Approximately 1 X 10⁶ cortical neurons were cultured on rectangular L-polylysine-coated coverslips and loaded with lysocerebroside or lysosphingomyelin to a level of 150 ± 20 pmoles per mg protein. The neurons were returned to complete Ham's high glucose medium/DMEM (1:1, vol:vol) and infiltrated with 5 µM fura 2/AM (Molecular Probes, Eugene, OR). Fura 2 is a calcium chelator whose fluorescence absorbance shifts to shorter wavelength upon calcium ion binding is measurable in intact cells. A change in intraneuronal calcium ion concentration was monitored as a change in the fluorescence spectrum.

The neurons were exposed to 10 µM glutamate agonist (for example, ibotenate) for 30 minutes at 37°C. The coverslips were then transferred to quartz cuvettes in medium containing 250 µM sulfinpyrazone to prevent fura 2/AM diffusion. Fluorescence ratios were recorded in a Perkin-Elmer LS-5 Fluorescence Spectrometer thermostatted at 37°C at excitation wavelengths of 340 and 380 nm, and emission measurement at 500 nm. Calcium ion signalling in agonist-stimulated neurons was 3.0 ± 0.2 arbitrary units for ibotenate-exposed neurons. In lysosphingomyelin and lysocerebroside-loaded neurons, agonists elicited no calcium signalling measurably over controls, indicating that agonist activity can be effectively blocked by inhibitory lysosphingolipids.

### Example 6: Monitoring Phospholipase C Activity in the Presence of Lysosphingolipids

### A. Monitoring free Phospholipase C Activity in Solution

The effects of sphingosine, lysocerebroside, and lysosphingomyelin on the kinetics of pure, immunologically isolated phospholipase C isoforms beta, delta, and gamma (which are commercially available) was examined. For each isoform, 10 µM sphingosine was added to a reaction medium consisting of 1 µg phospholipase C isoform/ml, Tris-malate buffer (50 mM, pH 7.0) containing 100 µM phosphatidyl inositol bis-phosphate tri-ammonium salt, 0.01 µCi tritium labeled phosphatidyl inositol bis-phosphate, 100 mM NaCl, 10 mM CaCl₂, and 5 mM 2-merceptoethanol. The sphingosine induced an approximately 2-fold increase in Vₘₐₓ, a decrease in calcium ion concentration required for optimal activity, and no effect on K_{M} for substrate phosphatidylinositol bis-phosphate. Conversely, 10 µM lysocerebroside or 5 µM lysosphingomyelin induced a 60% ± 10% diminution in Vₘₐₓ, a 5-fold increase in calcium ion concentration required for optimal activity, and no change in K_{M}. These observations indicate that the lysosphingolipids affect the ability of calcium to activate phospholipase C and operate on a regulatory domain of the enzyme.

### B. Monitoring Phospholipase C Activity in Intact Membranes

Physiologically active phospholipase C is present on the endofacial portion of the plasma membrane. A procedure for examining its activity in an intact membrane is provided. The neuronal membrane preparation described below is useful for determining lysosphingolipid effects on the phosphatidylinositol kinase and phospholipase C activities in intact membranes. One hundred Petri dishes of cultured cortical neurons were incubated overnight with 50 µCi [³H]myo-inositol per mL of culture medium. Ten Petri dish-samples of cultured neurons (1 X 10⁸ neurons; 23 ± 2 mg protein, n = 15) were pooled to produce 2.4 ± 0.2 mg plasma membrane by the following procedure. The collected neurons were homogenized in 5 mL 0.32 M sucrose and centrifuged at 1000 X g for 15 minutes. The supernatant was layered on top of 2 ml 1.2 M sucrose and centrifuged at 300,000 X g for 20 minutes. The sedimented pellet was resuspended by sonication for 10 seconds in 50 mM Tris buffer (pH 7.4), containing 0.3 µM CaCl₂, 1 mM MgCl₂, and 0.01% ascorbic acid.

The suspended plasma membrane preparation was apportioned into 250 µl samples, each providing approximately 100 µg membrane protein. Sphingosine, psychosine, and lysosphingomyelin were added to each plasma membrane sample. The samples were held at room temperature for 3 hours, centrifuged at 300,000 X g for 30 minutes, and the supernatant discarded. Samples were analyzed for lysosphingolipid loading (*i.e.*, membrane lysosphingolipid content following exposure to exogenous sphingolipid) by fluorescence tagging. Table 2 provides the results of exposure of plasma membrane samples to 10 µM lysosphingolipids. Values are reported in pmoles lysosphingolipid/mg plasma membrane protein. Values obtained for isolated plasma membrane and intact neurons are compared.

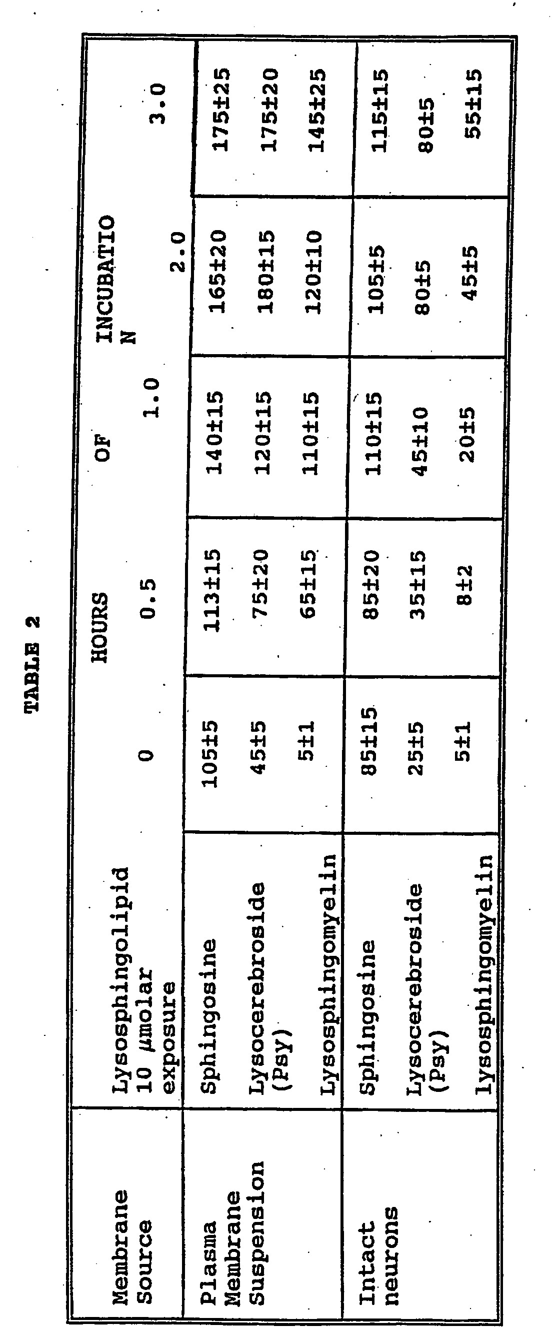

Phospholipase C activity in plasma membrane samples in the presence of neuronal agonists is determined as follows. Replicate series of control and lysophingolipid-loaded neuronal plasma membrane preparations pre-labelled with [³H]myo-inositol are analyzed for phosphatidylinositol phosphate hydrolysis and inositol phosphate release. Membrane samples equivalent to 100 µg membrane protein in 250 µl buffer (50 mM Tris buffer (pH 7.4), containing 0.3 µM CaCl₂, 1 mM MgCl₂, 0.01% L-ascorbate, and 1 µM ATP) are incubated for 30 minutes at 37°C in various concentrations of the neuronal agonists glutamate (0-100 µM), ibotenate, or quisqualate. Reactions are terminated by the addition of 1 ml ice-cold methanol to each sample followed by addition of 2 ml ice-cold chloroform. Diminution in phosphatidylinositol phosphates and release of inositol phosphates is estimated by thin layer chromatographic analysis, as described for phosphoinositidyl kinase activity analysis. Inositol phosphate release is plotted against µg lysosphingolipid per mg protein in the plasma membrane preparations and analyzed by Fisher's Least Squares Difference test. These data estimate lysosphingolipid modulation of phospholipase C activity in neuronal plasma membrane.

### C. Monitoring Phosphatidylinositol Kinase Activity in Neuronal Plasma Membrane

Candidate compounds for use in modulating calcium ion levels in cells via InsP₃ production are evaluated in terms of the enzymatic process they affect. The enzymes phosphatidylinositol kinase and phospholipase C both transfer from the cytoplasm to the endofacial lipid bilayer of the plasma membrane for physiological activity. To control for any affects of phosphatidylinositol kinase activity in the plasma membrane preparations, a procedure for monitoring activity of this enzyme is provided herein along with a procedure for monitoring phospholipase C activity. Also, procedures for monitoring the activities of these enzymes in the presence of candidate compounds is provided below. In the following examples, naturally occurring lysosphingolipids are tested.

Phosphatidylinositol kinase (control) and lysosphingolipid-loaded neuronal plasma membrane samples are used to test for phosphatidylinositol kinase activity. The content of lysosphingolipids was increased (in the lysosphingolipid-loaded membrane) by exposure to exogenous lysosphingolipids and each sample contained the equivalent to 100 µg membrane protein. The membrane samples are pelleted and resuspended in 250 µl of buffer (50 mM Tris buffer (pH 7.4) containing 0.3 µM CaCl₂, 1 mM MgCl₂, and 0.01% ascorbic acid) by sonication for 5 seconds. They are then cooled to 0°C and [³²P]-ATP (DuPont NEN, 1 TBeq/mmole) is added to 1 µM. The samples are then incubated at 20°C for 1 minute and placed in an ice bath to halt phosphorylation. The samples are vortexed with 1 ml ice-cold methanol:concentrated HCl (20:1, vol:vol). Two ml of ice-cold chloroform and 1 ml H₂O are added with continuous vortexing. The samples are centrifuged at 2000 X g for 10 minutes and the lower phase is drawn off.

Samples are analyzed for phosphatidylinositol and its mono- and bis-phosphate derivatives as a measure of phosphatidyl inositol kinase activity. Aliquots of each test sample, as well as reference standards of phosphatidylinositol and the mono- and bis-phosphate derivatives (Sigma Chemical Co., St. Louis, MO), are assayed, for example, by thin layer chromatography. Aliquots are chromatographed on silica gel G thin layer plates (Merck) that have been pre-soaked in 1% potassium oxalate dissolved in methanol:water (1:1, vol:vol), and dried at 120°C for 1 hour. The plates are developed with the following mixture of solvents: chloroform:methanol:acetic acid:water at 40:15:12:13:8 (vol:vol). The developed plates are sprayed with Molybdenum Blue reagent (Sigma) which visualizes lipid-bound phosphoryl groups. Phosphatidylinositol, phosphatidylinositol mono-phosphate, and phosphatidylinositol bis-phosphate contents are analyzed relative to membrane protein by scanning in a BioRad Videodensitometer Model 620 coupled with the BioRad 1-D analyst program. Bands are then scraped and analyzed for radioactivity in a Beckman LS 5800 Scintillation Spectrometer.

Control and lysosphingolipid-loaded plasma membrane activities are compared by plotting phosphorylation labelling at t_{1/2} (1 minute) against pg lysosphingolipid per mg membrane protein. Data are analyzed by Fisher's protected least significant difference test.

### Example 7: Methods of Modulating Inositol Trisphosphate Production in a Neuron in vivo for Treatment of a Neuronal Disorder

Inositol trisphosphate production is modulated *in vivo* by administering a compound, such as a naturally occurring lysosphingolipid, to a mammal exhibiting symptoms of a neuronal disorder associated with aberrant InsP₃ production. Disorders associated with undesirable increases in InsP₃ productian have been reviewed above and include acute brain hypoxia/ischemia and brain seizure, while chronic disorders include Alzheimer's disease, Huntington's disease, and amyotrophic lateral sclerosis. A disorder associated with an undesirable decrease in InsP₃ production is neuronal apoptosis or programmed cell death. Administration of the compound is performed under conditions such that symptoms of the disorder are controlled to a desirable level or alleviated.

A candidate compound for use in modulating InsP₃ production *in vivo* is screened by administering the candidate compound to an animal exhibiting a neuronal disorder associated with aberrant InsP₃ production. An example of such a test animal includes, but is not limited to, a DBA/2 mouse having audiogenic seizures (Thomsen *et al*., J. Neurochem. 62:2492-2495, 1994; incorporated by reference specifically to include a method of administering a neuroactive compound to an animal). Another *in vivo* screening model uses a gerbil which has surgically occluded common carotid arteries, which produces brain ischemia/anoxia (Matesik *et al*., J. Neurochem., 63:1012, 1994). Also incorporated by reference as *in vivo* screening models are the focally ischemic rat model (Zobrist *et al*., Stroke, 24:2002, 1994) and the Alzheimer's disease model adult mouse treated with beta-amyloid peptides (Hartmann *et al*., Biochem. Biophys. Res. Commmun., 194:1216, 1993).

### Example VIII. In vivo Studies

Application of the specific metabotropic glutamate receptor agonist, quisqualate, to the lateral ventricle of the brain produces a dose-dependent behavioral response and loss of hippocampal neurons in rats. At a high dose (250 nmoles per animal), quisqualate produced severe convulsions and death. At a moderate dose (225 nmoles per animal), quisqualate produces moderate convulsions and death in 60% of animals tested. At a low dose (150 nmoles per animal), animals exhibit an increase in activity and teeth chattering. Seven days after application, both the moderate and high doses of' quisqualate produce severe loss of neurons in the hippocampus. When these animals were pretreated with 125 nmoles of lysocerebroside (psychosine) or lysosphingomyelin, the behavioral and histological changes caused by quisqualate were absent. From this it was concluded that lysocerebroside or lysosphingomyelin are capable of prevent metabotropic glutamate excitotoxicity *in vivo*. In addition, no behavioral or neurotoxic effects were observed by doses of these compounds as high as 125 nmoles per animal.

The animals used in this experiment were male F344 rats (Charles River Breeding Laboratories). The animals were housed individually, exposed to a 12-hour light-dark cycle, and allowed free access to food and water. The animals weighed approximately 250 g at the beginning of each experiment and were weighed daily. In addition, the animals were habituated to handling before each experiment.

Before administering various compounds, the animals were anesthetized with sodium pentobarbital (50 mg/kg, i.p.) and positioned in a sterotaxic instrument. A 26 gauge cannula was inserted into the cerebral ventricle at the following co-ordinates: 1.0 mm posterior to bregma, 1.5 mm lateral to the midline, and 4.0 mm below the dorsal surface of the neocortex. Cannulas were capped and fixed in place with dental cement. One week after cannulation, 10 µl of each of the treatment drugs was infused, ICV (i.e. into the ventricals of the brain), via a micropump at a rate of 10 µl/min. For this procedure, the animals were lightly restrained and unanesthetized. Following administration of the compound, the infusion cannula remained in position for one minute, after which the permanent *in situ* cannula was capped.

The compounds administered were obtained from a commercial supplier (Sigma Chemical Co., St. Louis, MO) and dissolved in saline prior to injection. These compounds included quisqualate (injected at doses ranging from 100 nmole/10 µl to 1 µmole/10 µl), lysocerebroside, and lysophingomyelin (which were injected at doses of 25 nmoles/10 µl to 150 nmoles/10 µl).

### A. Histological Analysis

Seven days after the compounds were administered, animals were sacrificed by transcardial perfusion with neutralized, buffered 10% formalin-saline. Their brains were removed, post-fixed overnight in 10% formalin-saline, and sectioned along the coronal plane (into 50 µm thick sections) on a freezing microtome. The sections were stained with cresyl violet to determine the extent of cellular destruction.

A subset of the animals in each group was sacrificed by transcardial perfusion with 4% paraformldehyde. The brains of these animals were removed, post-fixed for 48 hours in 4% paraformaldehyde, placed in PBS, embedded in paraffin, and sectioned at room temperature into 6 µm thick setions along the coronal plane.

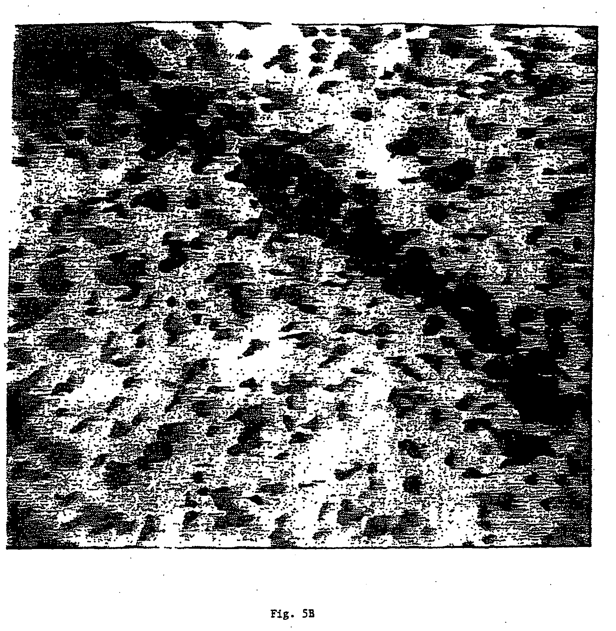

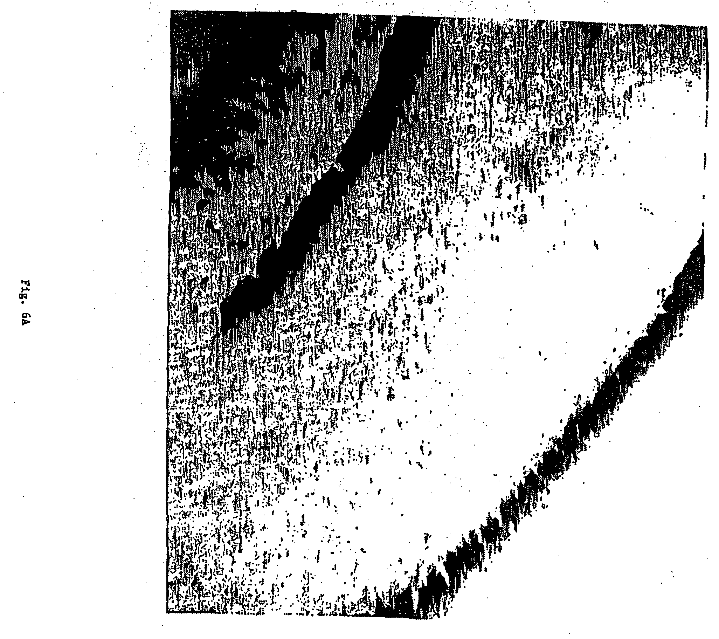

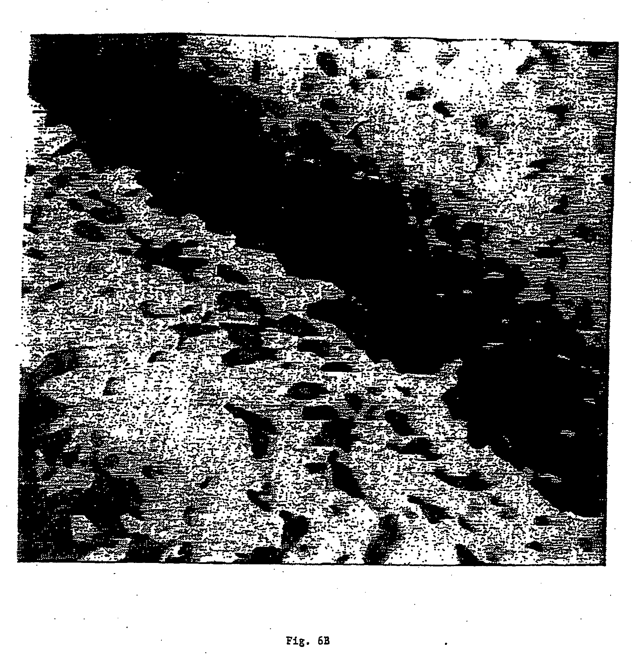

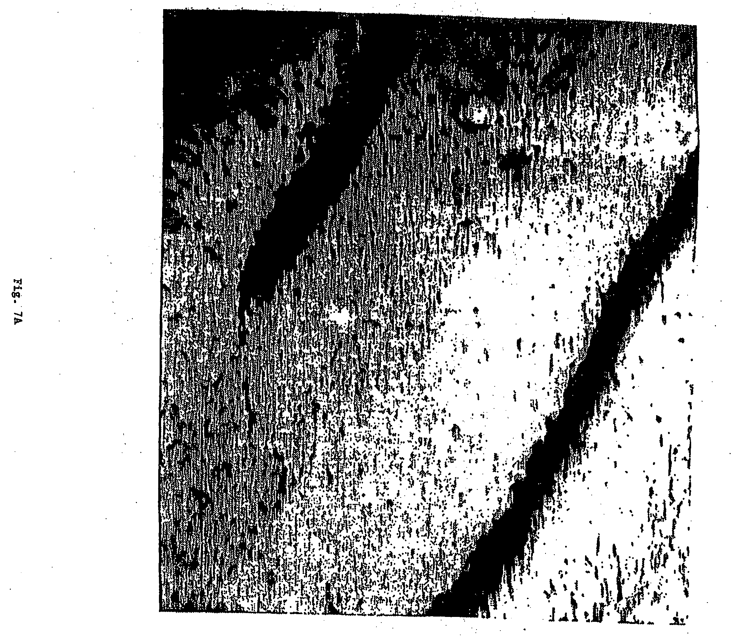

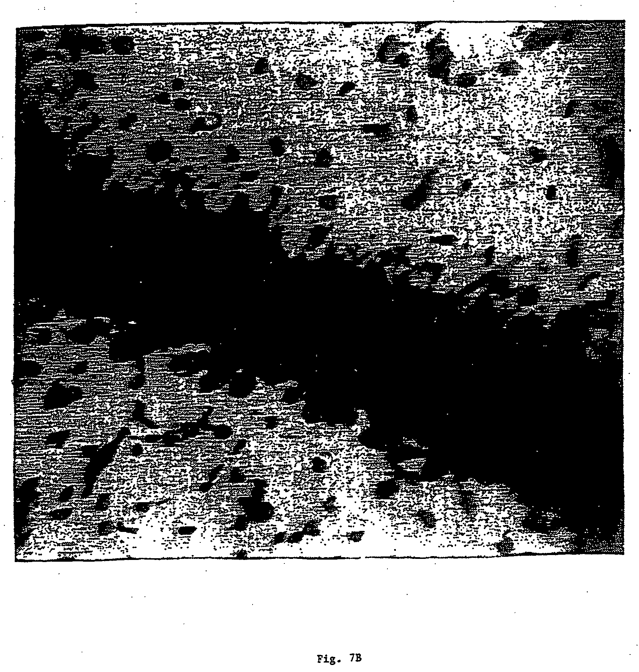

Given that high concentrations of metabotropic receptors are located in the hippocampus, and that the hippocampus is particularly vulnerable to the effects of a variety of insults including ischemia and hypoxia, this region was examined for evidence of neuronal degeneration due to glutamate excitoxicity. Histological analysis of brain slices using cresyl violet staining confirm that quisqualate (225 nmoles) injected ICV produces a considerable loss of CA1 pyramidal neurons in the hippocampus (Figs. 5A and 5B) compared to saline-injected controls (Figs. 6A and 6B) and that pretreatment with psychosine (125 nmoles) prevents the neuronal loss caused by quisqualate (Figs. 7A and 7B).

### B. Behavioral Analysis

Pilot studies identified several specific behavioral measures that could be used to indicate excessive activation of the metabotropic receptor (see Table 3). These behaviors include convulsions, spasms, teeth chattering, akinesia, and motor activity. The experiments that follow were based on an analysis of these behaviors over a two hour period after administration of the compounds of the invention. Behavioral observation was carried out by two trained observers, who worked independently of one another and who were blind to the experimental groups. The frequency of convulsions and spasms, and the duration of teeth chattering, akinesia and mobilizing was recorded continuously for 20 minutes prior to administration of the compound of the invention and for 2 hours after the treatment. Frequency and duration means were computed by subtracting pre-infusion means from post-infusion means.

Some of the experiments that were performed first were carried out to determine the amount of quisqualate required to produce behavioral and histological changes and the amount of lysosphingolipids that could effectively prevent or reverse these effects. Table 3 outlines the behavioral responses of rats to various doses of quisqualate (injected as described above) and the associated histological changes. The behaviors are listed as they occur chronologically. The limb clasp and reflex tests indicate the degree of decortication. A limb clasp or placement score of zero is normal, and a score of 3 is severely abnormal.

**Table 3**

| **DOSE** Quisqualate | **BEHAVIOR** | **HISTOLOGICAL CHANGES** |
|---|---|---|
| **500 µmoles** | vocalization | loss of neurons in the piriform and entorhinal cortex. CA1 and dentate gyrus regions of the hippocampus |
| **1 µmoles** | -severe repeated full body convulsions | as above |
| | -severe scratching | |
| | -severe teeth chattering | |
| | -foaming at mouth | |
| | -dead 5-15 mins in 100% cases | |
| **250 nmoles** | -vocalization | as above |
| | -teeth chattering | |
| | -scratching | |
| | -localized spasms jerking/twitching | |
| | -full body convulsions-severe/repeated | |
| | -foaming at mouth | |
| | -respiratory distress | |
| | -abnormal placement and clasp reflex (3) | |
| | -death in 75% of cases | |
| **225 nmoles** | -teeth chatter | loss of CA1 pyramidal neurons in the hippocampus |
| | -hind leg paralysis | |
| | -hyperactivity/walk in circles | |
| | -full body convulsions - moderate | |
| | -localized spasms twitching/jerking | |
| | -episodic akinesia/tonic immobility | |
| | -respiratory distress | |
| | -foaming at mouth | |
| | -abnormal placement and clasp reflex (1-2) | |
| | -death in 60% of cases | |
| **200 nmoles** | -teeth chatter | as above |
| | -occasional mild convulsions | |
| | -hyperactivity - circle walking | |
| | -episodic akinesia/tonic immobility | |
| | -hind leg weakness | |
| | -normal limb clasp and placement reflex (0) | |
| **150 nmoles** | -teeth chatter | none observed |
| | -localized twitches | |
| | -mild hyperactivity walking in circles | |
| | -hind leg weakness for 60 minutes | |
| | -normal placement and clasp reflex (0) | |
| **100 nmoles** | -teeth chatter | none observed |
| | -mild scratching | |
| | -hind leg weakness 60 minutes | |
| | -normal placement and clasp reflex (0) | |

The effective dose of quisqualate was determined to be 225 nmoles, that of lysocerebroside was determined to be 125 nmoles, and that of lysosphingomyelin was determined to be either 125 nmoles or 150 nmoles (the higher doses appeared to be more reliable in pilot testing).

The data shown in Table 4 represents the observations of pilot studies aimed at determining the effect (on behavior) of lysosphingolipids infused directly into the lateral ventricles of the brain 30 minutes prior to infusion of quisqualate.

**TABLE 4**

| **DOSE** | **BEHAVIORAL RESPONSE** |
|---|---|
| Psychosine 25 nmoles + Quisqualate 200 moles | Moderate convulsions, teeth chattering, akinesia and hyperactivity |
| Psychosine 50 nmoles + Quisqualate 200 moles | Moderate convulsions, teeth chattering, akinesia and hyperactivity |
| Psychosine 100 nmoles + Quisqualate 200 nmoles | Almost complete attenuation of Quisqualate response, some teeth chattering post-injection |
| psychosine 125 moles + Quisqualate 200 moles | Complete attenuation of Quisqualate response |
| Psychosine 125 nmoles + Quisqualate 225 nmoles | Complete attenuation of Quisqualate response |
| Psychosine 100 nmoles + Quisqualate 250 nmoles | Moderate convulsions, teeth chattering, akinesia. Quisqualate markedly reduced compared to typical Quisqualate response at this dose. |
| Psychosine 150 nmoles + Quisqualate 250 nmoles | Almost complete attenuation of Quisqualate response. Psychosine produced short term sedation at this dose. |
| Lysosphingomyelin 100 moles + Quisqualate 225 nmoles | Almost complete attenuation of Quisqualate response, some teeth chattering post-injection and hyperactivity. |
| Lysosphingomyelin 150 nmoles + Quisqualate 225 nmoles | Complete attenuation of quisqualate response. Lysosphingomyelin produced mild sedation at this dose. |

The experiment began one week after ICV cannulation. Animals (n = 6 per group) were randomly allocated to one of 8 treatment groups: (1) saline, (2) quisqualate at 225 nmoles, (3) psychosine at 125 nmoles, (4) lysosphingomyelin at 125 nmoles, (5) lysosphingomyeliri at 150 nmoles, (6) quisqualate at 225 nmoles and psychosine at 125 nmoles, (7) quisqualate at 225 nmoles and lysosphingomyelin at 125 nmoles, and (8) quisqualate at 225 nmoles and lysosphingomyelin at 150 nmoles. Following the baseline observation period, animals received the first of two ICV infusions (all infusions were of a 10 µl volume that was administered over a 1 minute period). For the first five groups listed above, this consisted of saline (10 µl/minute), for the following 3 groups the infusions were psychosine at 125 nmoles, lysosphingomyelin at 125 nmoles, or lysosphingomyelin at 150 nmoles, respectively. Thirty minutes later, all animals received the second infusion which, for the first five groups listed above, consisted of saline, quisqualate at 225 nmoles, psychosine at 125 nmoles, lysosphingomyelin at 125 nmoles, and lysosphingomyelin at 150 nmoles respectively. The remaining three groups received infusions of quisqualate at 225 nmoles. Following the second infusion animals were returned to their cages, and observed continuously over the subsequent 2 hours.

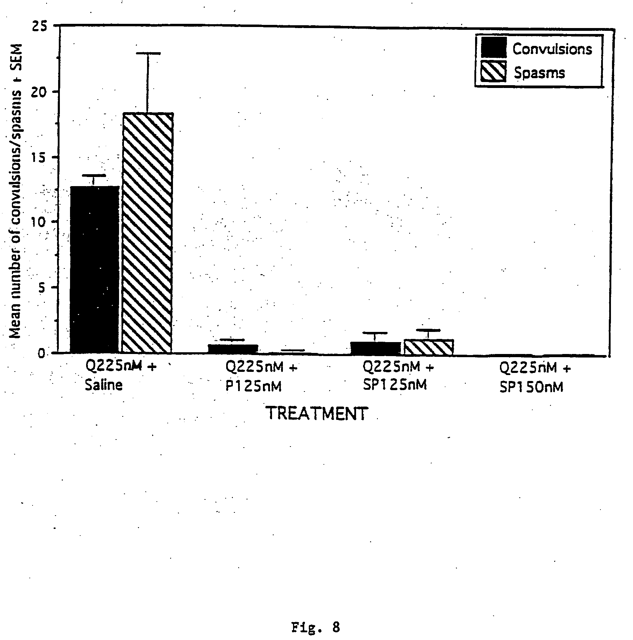

During the observation period, two behavioral analyses were carried out. The first on the frequency of spasms and convulsions, the second on the duration of teeth chattering, akinesia and motor activity. Figure 8 illustrates the mean number of convulsions or spasms across 4 of the main treatment groups (quisqualate at 225 nmoles + saline; quisqualate at 225 nmoles + psychosine at 125 nmoles; quisqualate at 225 nmoles + lysosphingomyelin at 125 nmoles; and quisqualate at 225 nmoles + lysosphingomyelin at 150 nmoles). The remaining 4 control groups (saline; psychosine at 125 nmoles; and lysosphingomyelin at 125 nmoles and 150 nmoles) have not been illustrated, since animals in these treatment groups displayed no convulsant behavior. A two way ANOVA, group X response on the mean frequency of convulsions or spasms revealed a significant group effect (F(7.24=9.75, p .05). Multiple comparisons, using the Bonferroni adjustment, revealed that there were significantly (p.<01) more convulsions/spasms in the group injected with quisqualate alone at 225 nmoles compared to all other groups. Pretreatment with Psychosine (125 nmoles) or Lysosphingomyelin (125 nmoles and 150 nmoles) significantly (p<01) attenuated the number of convulsions or spasms, although there was no significant difference between the three treatment groups.

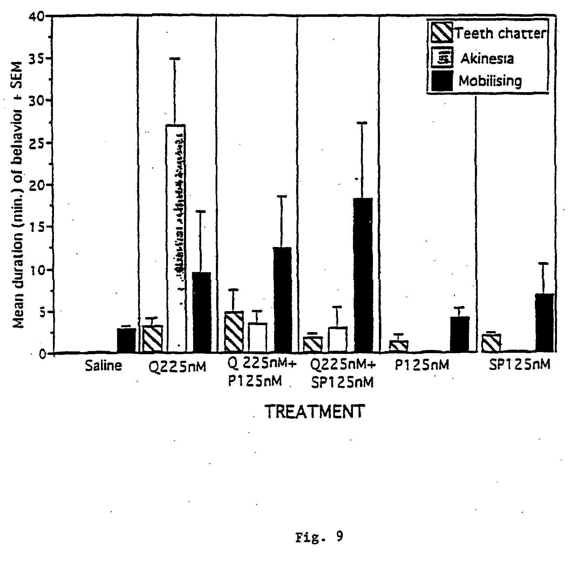

The second analysis was carried out on the duration of teeth chattering, akinesia and mobilization. Fig. 9 illustrates the mean duration of each of these behaviors. A two way ANOVA, group X behavior, revealed a significant group effect (F(7.34)=4.66, p=0.01) and a significant behavior effect (F(2.68)=3.98, p=0.023). Multiple comparisons, using the Bonferroni adjustment, on the group effect revealed that animals injected with quisqualate alone spent significantly (p< .01) more time engaged in these behaviors than did animals injected with the lysophingolids alone (psychosine at 125 nmoles and lysophingomyelin at 125 nmoles and 150 nmoles) and animals injected with quisqualate 225 nmoles and lysophingomyelin 150 nmoles. Multiple comparisons on the behavior effect revealed that animals spent significantly (p< .05) more time mobilizing compared to engaging in teeth chatter.

Although the invention has been described with reference to the presently preferred embodiments, it should be understood that various modifications can be made without departing from the spirit of the invention.

## Claims

1. Use of a composition comprising a compound which decreases inositol trisphosphate concentration by acting downstream from a cell surface receptor, for the preparation of a pharmaceutical composition for decreasing inositol trisphosphate concentration in a neuronal cell of a mammal having or suspected of having a disorder associated with an abnormal concentration of inositol trisphosphate, wherein the disorder is brain anoxia, brain ischemia, brain seizure activity, or a chronic neurodegenerative disorder including Alzheimer's Disease, Parkinson's Disease, Huntington's Disease, or amyotrophic lateral sclerosis.

2. The use of claim 1, wherein the compound is lysocerebroside or lysosphingomyelin.

3. The use of claims 1 or 2, wherein the neuron is a central nervous system neuron peripheral nervous system neuron.

4. The use of claim 3, wherein the neuron is a brain neuron.

5. An in vitro method of identifying a compound which decreases neuronal inositol trisphosphate concentration, the method comprising:
a) contacting a neuron with inositol and a compound sufficient to modulate inositol trisphosphate production; and
b) measuring the amount of inositol trisphosphate produced.

6. The method of claim 5, wherein the compound modulates a decrease in inositol trisphosphate.

7. The method of claim 5, further comprising contacting the neuron with a second compound that modulates inositol trisphosphate concentration.
